(19) Europäisches Patentamt
European Patent Office
Office européen des brevets

(11) **EP 4 685 756 A1**

(12) **EUROPEAN PATENT APPLICATION**

(43) Date of publication:
**28.01.2026 Bulletin 2026/05**

(21) Application number: **25166487.6**

(22) Date of filing: **26.03.2025**

(51) International Patent Classification (IPC):
**G06V 10/82** *(2022.01)* **G16H 30/40** *(2018.01)*
**G16H 50/20** *(2018.01)*

(52) Cooperative Patent Classification (CPC):
**G06V 10/82; G16H 30/40; G16H 50/20**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **24.07.2024 PT 2024119615**

(71) Applicants:
• **INESC TEC - Instituto de Engenharia de Sistemas e Computadores, Tecnologia e Ciência**
**4200-465 Porto (PT)**
• **Universidade Do Porto**
**4099-002 Porto (PT)**

(72) Inventors:
• **NUNES FERREIRA, CARLOS ALEXANDRE**
**4200-465 PORTO (PT)**
• **RIBEIRO FERREIRA, ALEXANDRE**
**4200-465 PORTO (PT)**
• **PINHEIRO DA SILVA GUIMARÃES, NUNO RICARDO**
**4200-465 PORTO (PT)**
• **TAVARES COIMBRA, MIGUEL**
**4200-465 PORTO (PT)**
• **GUEDES JORGE, ALÍPIO MÁRIO**
**4200-465 PORTO (PT)**
• **DE CASTRO CAMPILHO, AURÉLIO JOAQUIM**
**4200-465 PORTO (PT)**

(74) Representative: **Patentree**
**Edificio Net**
**Rua de Salazares, 842**
**4149-002 Porto (PT)**

(54) **METHOD AND DEVICE FOR DETECTING AND ANNOTATING LESIONS IN A MEDICAL IMAGE, BASED ON A MEDICAL DESCRIPTION**

(57) A computer-implemented method and data processing device for detecting lesions from an image obtained by medical imaging and from a medical description, comprising: obtaining identified entities and corresponding attributes from the medical description; obtaining candidate lesions and corresponding attributes from the image obtained by medical imaging; matching the candidate lesions and corresponding attributes with the identified entities and corresponding attributes; and selecting the matched filtered candidate lesions and identified entities using a highest probability criterion corresponding to an entity identified in the medical description matching a candidate lesion obtained from the medical image.

Fig. 2B

## Description

### TECHNICAL FIELD

The present disclosure relates to a computer-implemented method for spatially annotating a pre-described medical image, in particular a pulmonary image.

### BACKGROUND

The state of the art in image annotation and detection using text involves several cutting-edge technologies and models that integrate natural language processing (NLP) with computer vision.

Segment Anything Model (SAM): Developed by Meta's FAIR lab, SAM is designed for promptable segmentation, where a prompt can be anything from foreground/background points to free-form text. This model can predict segmentation masks based on these prompts, enabling zero-shot transfer to various tasks. SAM's robustness is partly due to the extensive SA-1B dataset, which contains over a billion high-quality segmentation masks. SAM's versatility makes it applicable in fields like AI-assisted labelling, medical imaging, and environmental monitoring (Kirillov et al., 2023).

CLIP (Contrastive Language-Image Pre-Training): CLIP, developed by OpenAI, is a foundational model that correlates images with text descriptions. It performs zero-shot learning for various image classification and annotation tasks by understanding the relationship between text and images. CLIP's capabilities are significant for automatic image annotation, especially in scenarios requiring detailed and context-aware descriptions (Radford et al., 2021).

YOLO-World: Introduced in early 2024, YOLO-World is a significant advancement in real-time open-vocabulary object detection. It extends the capabilities of traditional YOLO models by incorporating a vision-language approach, enabling the detection of objects beyond predefined categories. YOLO-World uses a new architecture called the Reparameterizable Vision-Language Path Aggregation Network (RepVL-PAN), which enhances the interaction between visual and linguistic information. This model supports zero-shot object detection by leveraging large-scale pre-training on image-text datasets, achieving high accuracy and speed on benchmarks like the LVIS dataset. The prompt-then-detect paradigm employed by YOLO-World allows for efficient and flexible object detection without extensive real-time computation (Cheng et al., 2024).

However, existing approaches often face limitations in accurately correlating textual medical descriptions with spatial image annotations, particularly in scenarios where predefined models such as SAM, CLIP, or YOLO-World are not directly applicable. Methods relying on direct segmentation or object detection struggle with complex spatial references, variable terminology, and the absence of directly prompted data in clinical reports.

These facts are disclosed in order to illustrate the technical problem addressed by the present disclosure.

### GENERAL DESCRIPTION

The present disclosure describes a method for spatially annotating a previously described medical image in text format, in particular a pulmonary image, by mapping specific parts of the description to corresponding regions within the medical image, for example a CT scan image or an MRI scan image.

In an embodiment, the method firstly processes parts of the text, such as identifying and annotating specific medical conditions like lung nodules. This application highlights the potential for targeted image annotation based on selective text processing, making automatic the image annotation.

As disclosed above, existing approaches often face limitations in accurately correlating textual medical descriptions with spatial image annotations, particularly in scenarios where predefined models such as SAM, CLIP, or YOLO-World are not directly applicable. Methods relying on direct segmentation or object detection struggle with complex spatial references, variable terminology, and the absence of directly prompted data in clinical reports. In contrast, according to the present disclosure, modular and 3D-aware solutions are shown to be able to provide a more flexible framework by leveraging independent components that can process and integrate textual and visual cues.

It is thus disclosed a computer-implemented method for detecting and annotating lesions from an image obtained by medical imaging, and based on a corresponding medical description comprising identified entities of medical lesions, i.e. a clinical report, the method comprising the steps:

> obtaining identified entities and corresponding attributes from the medical description;
> obtaining candidate lesions and corresponding attributes from the image obtained by medical imaging;
> matching the candidate lesions and corresponding attributes with the identified entities and corresponding attributes; and
> selecting the matched filtered candidate lesions and identified entities using a highest probability criterion corresponding to an entity identified in the medical description matching a candidate lesion obtained from the medical image.

An embodiment comprises corresponding attributes of an entity identified in the medical description with matching attributes of a candidate lesion obtained from the medical image, wherein said attributes comprise size.

An embodiment comprises integrating [i.e. calculating an integral of] a probability of a voxel of a candidate lesion in the medical image matching an entity identified in the medical description.

In an embodiment, selecting the matched filtered candidate lesions and identified entities using a highest probability criterion is independently carried out for each anatomical region of a plurality of anatomical regions present in the medical image and/or present in the medical description.

In an embodiment, the plurality of anatomical regions is obtained by segmentation of the medical image.

In an embodiment, obtaining the identified entities and corresponding attributes from the medical description, comprises the steps of:

extracting token embeddings from the medical description using an encoder-only transformer model;
extracting feature embeddings from the medical description using a character convolutional neural network model;
identifying entities and respective attributes, said attributes comprising a location of each identified entity, from the extracted token embeddings and from the extracted feature embeddings using a bidirectional long short-term memory neural network followed by a conditional random field model;
extracting text segments from the medical description for each of the identified entities, where the text segment includes the identified entity;
extracting token embeddings from the extracted text segments using an encoder-only transformer model and tagging the identified entities with a tagging model to classify relationships between said attributes; and
pairing the identified entities using a probability model.

In an embodiment, the tagging of the identified entities with a tagging model comprises assigning attributes that denote the beginning, inside, or outside of a text segment encompassing an identified entity.

In an embodiment, obtaining candidate lesions and corresponding attributes from the image obtained by medical imaging, comprises the steps of:

segmenting the medical image into image segments, wherein the image segments comprise the locations of the identified entities;
obtaining candidate lesions and corresponding attributes from the image segments using a convolution neural network; and
filtering candidate lesions using a false positive reduction model for removing false positive candidate lesions.

In an embodiment, a lesion is a damage or abnormal change in a tissue.

In an embodiment, the attributes of the identified entities from the medical description comprise at least one of: a first location related to lesion location (or Absolute Location); a second location related to lesion relative location (or Relative Location); a lesion type; a lesion size attribute; anatomical lesion characteristic or characteristics; a lesion quantity attribute; and/or a non-appearance attribute.

In an embodiment, the encoder-only transformer model is a Bidirectional Encoder Representations from Transformers (BERT) model.

It is also disclosed a data processing device configured for carrying out the steps of the method according to any of the disclosed embodiments.

It is also disclosed a computer program comprising instructions which, when the program is executed by a computer, cause the computer to carry out the steps of the method according to any of the disclosed embodiments.

It is also disclosed a computer-readable storage medium comprising instructions which, when executed by a computer, cause the computer to carry out the steps of the method according to any of the disclosed embodiments, in particular the computer-readable storage medium being a non-transitory computer-readable storage medium.

In an embodiment, the method starts with the input of the words from the text of a clinical report, where each word is transformed into a numerical vector that represents its meaning, i.e. an embedding. This is performed by a BERT large model. Next, a Character Convolutional Neural Network (CharCNN) takes the letters, i.e., characters, and sub words, e.g., prefixes or suffixes, of each word as input and generates a representation based on the detected patterns. The output of the CharCNN is combined with the embedded sequence of words and passed to the Bidirectional Long Short Term Memory (BiLSTM), which creates an embedding of each word considering the context of the entire sentence. Finally, this sequence of word representations is fed into a Conditional Random Field (CRF), which categorizes each word, taking into account the interactions between them in the sentence.

In an embodiment, a second BERT module receives as input the entire text document, the previously identified and categorized words, and any additional syntactic and grammatical information, to generate contextual embeddings.

This solution is particularly advantageous as it uses a small dataset for retraining; it is module-based which can be replaced and adapted if needed; and it is more interpretable and explainable than other approaches.

The identified problems in the state of the art are solved via a full automatization of the method, thus performing the annotation in a short time regardless of the quantity of images to annotate, and saving time during image (re)visitation by a medical doctor;

Additionally, inferences for the same model always yield the same results, being objective, and thus taking into account the textual content and image training history to provide what is most suitable.

This disclosure offers several key advantages, including: the no need for specialized professionals to generate large volumes of annotated data; the fact that image annotation is still a time-consuming and manual task performed by medical doctors; and the potential for varying interpretations of imaging findings when textual reports are ambiguous, as different clinicians may understand them differently.

## BRIEF DESCRIPTION OF THE DRAWINGS

The following figures provide preferred embodiments for illustrating the disclosure and should not be seen as limiting the scope of invention.

**Figure 1:** Flowchart representation of an embodiment for obtaining identified entities and corresponding attributes from the medical description.

**Figure 2A:** Flowchart representation of an embodiment for obtaining candidate lesions and corresponding attributes from the image obtained by medical imaging.

**Figure 2B:** Flowchart representation of an embodiment for matching the candidate lesions and corresponding attributes with the identified entities and corresponding attributes.

**Figure 3:** Schematic representation of an embodiment of the entity and respective attribute extraction.

**Figure 4:** Schematic representation of an embodiment of the computer vision method.

**Figure 5:** Schematic representation of an embodiment of the Named Entity Recognition (NER) method.

**Figure 6:** Schematic representation of some of the relations between recognized entities that are established in a relation extraction example sentence.

**Figure 7:** Schematic representation of an embodiment of the relation extraction method with [ST] as the start of the text and [ET] as the end of the text.

**Figures 8A and 8B:** Schematic representation of an embodiment of the automatic lung nodule detection method.

## DETAILED DESCRIPTION

The present document discloses a method and device for detecting and annotating lesions in a medical image, based on a medical description.

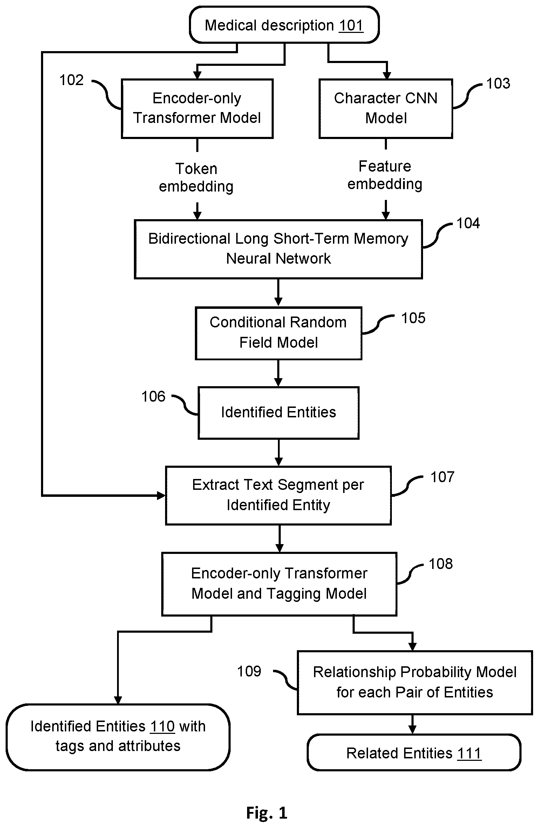

**Figure 1** shows a schematic representation of an embodiment of the annotation conversion to tabular data, wherein: **101** represents the medical description intended to be annotated with respect to pulmonary nodules; **102** represents the encoder-only transformer model; **103** represents the character CNN (charCNN) model; **104** represents the Bidirectional Long Short-Term Memory (BiLSTM) neural network; **105** represents the Conditional Random Field; **106** represents the identified entities; **107** represents the extract text segment corresponding to the identified entity; **108** represents the encoder-only transformer model and tagging; **109** represents the relationship probability model for each pair of entities; **110** represents the identified entities; and **111** represents the related entities.

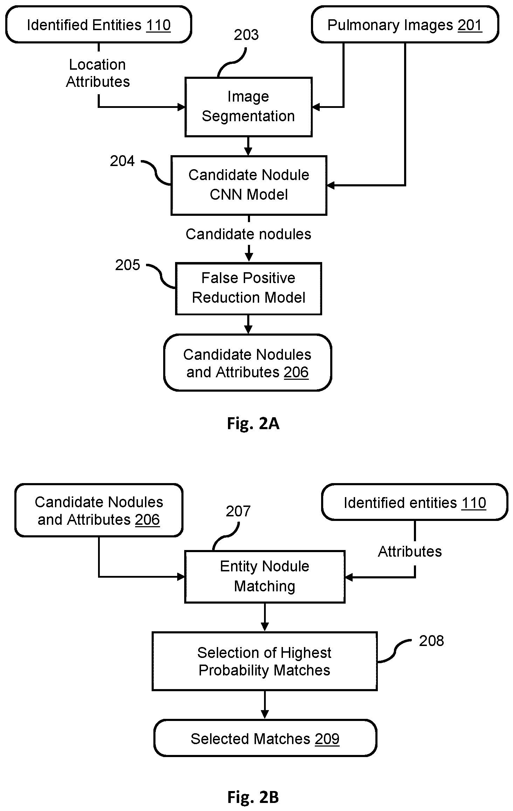

**Figure 2A** shows a schematic representation of an embodiment of the nodule candidate detection method in pulmonary images, more commonly referred to as anatomic segmentation and nodule detection stages of the Computer Vision (CV) method, wherein: **201** represents the pulmonary images; **110** represents the identified attributes obtained from identified entities (from **Figure 1**); **203** represents the anatomic segmentation method for location identification; **204** represents candidate nodule CNN model, which will output candidates for possible nodules; **205** represents the false positive reduction model, yielding a reduced number of candidate nodules; and **206** represents the aforementioned reduced number of candidate nodules, as well as the attributes from each of the respective candidate.

**Figure 2B** shows a schematic representation of an embodiment for matching the candidate lesions and corresponding attributes with the identified entities and corresponding attributes, wherein: **110** represents the identified attributes obtained from identified entities (from **Figure 1**); **206** represents the aforementioned reduced number of candidate nodules, as well as the attributes from each of the respective candidate; **207** represents the entity nodule matching; **208** represents the selection of highest probability candidate(s); and **209** represents the selected matches.

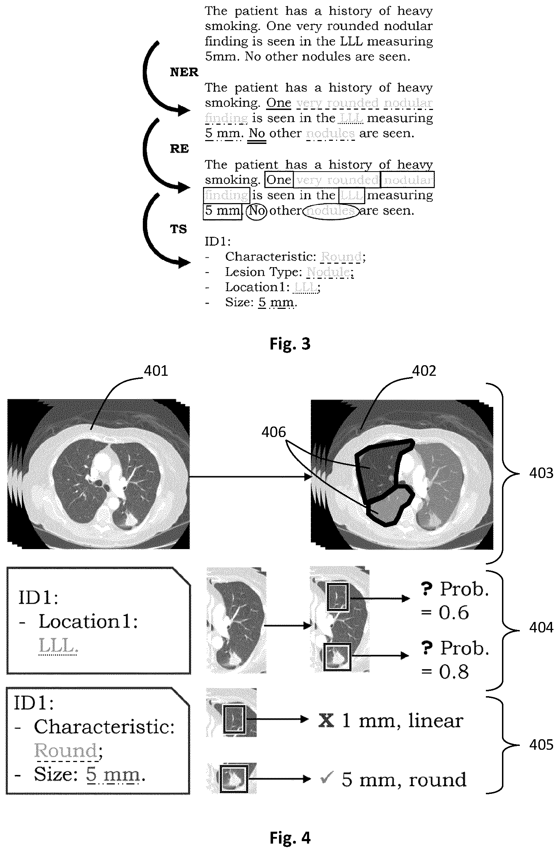

**Figure 3** shows a schematic representation of an embodiment of the method for entity and respective attribute extraction, wherein: solid line indicates a quantity; a dashed line indicates a characteristic; a dash/dot line indicates a lesion type; a dotted line indicates a first location; a double dot/dash line indicates a size; a double line indicates a negative; ID1 is represented by a rectangular shape; ID2 is represented by an ellipsoid shape; where NER stands for Named Entity Recognition, RE stands for Relation Extraction, and TS stands for Text Simplification.

**Figure 4** shows a schematic representation of an embodiment of the CV method, wherein **401** represents a set of input images, **402** represents a set of output images, **403** represents an anatomic segmentation, **404** represents a nodule detection, **405** represents a matching decision, and **406** represents an identified region. In **404,** "Prob." stands for a probability of the detected sub-image corresponding to a nodule.

The module for entity and respective attribute extraction, in an embodiment, is subdivided into three distinct submodules: NER, RE, and TS submodules. The goal of NER is to identify entity attributes, such as those already mentioned.

In an embodiment, the "uncertainty" attribute was not considered due to the limited number of cases; instead, expressions of "quantity" and "negative" were included. The "negative" attribute significantly alters the meaning of sentences, as in "No nodules are present." while the "quantity" attribute handles expressions like "...two nodules...". Consequently, the attributes considered, for this example, are:

Location1 (Absolute Location), Location2 (Relative Location), Lesion Type, Size, Characteristics, Quantity, and Negative.

In an embodiment, the RE submodule is responsible for grouping relationships between the entity attributes. For instance, RE can determine that a "nodule" is related to a specific "lung lobe", thereby structuring the information in a meaningful way.

For better results, the TS submodule focuses on reducing complex expressions to their simpler forms. For example, phrases such as "nodular finding", "slightly elongated contours", and "of considerable volume" are simplified to "nodule", "elongated", and "large". This simplification ensures that the extracted information is concise and standardized, facilitating further processing and analysis.

In an embodiment, the CV module is similarly divided into three submodules: Anatomic Segmentation, Nodule Detection, and Matching Decision. This module locates precisely the nodules referred to in medical reports, after the attributes and relationships between these attributes have been extracted. The anatomic segmentation submodule focuses on segmenting anatomical regions, such as lung lobes, to define the search area for potential pathological lesions.

This segmentation is advantageous, as it frames the regions of interest within specific anatomical positions (e.g., anterior/posterior, lateral/medial, superior/inferior), thus narrowing the search scope. The nodule detection submodule then identifies regions within the segmented area that exhibit features consistent with nodules. This involves analysing the segmented regions to pinpoint areas with a high likelihood of being nodules based on their characteristics. Finally, the matching decision submodule evaluates the detected candidates, comparing them against the characteristics extracted from medical texts. The candidate with the highest probability of fitting the required characteristics is selected accordingly, ensuring that the nodule mentioned in the text report is accurately identified.

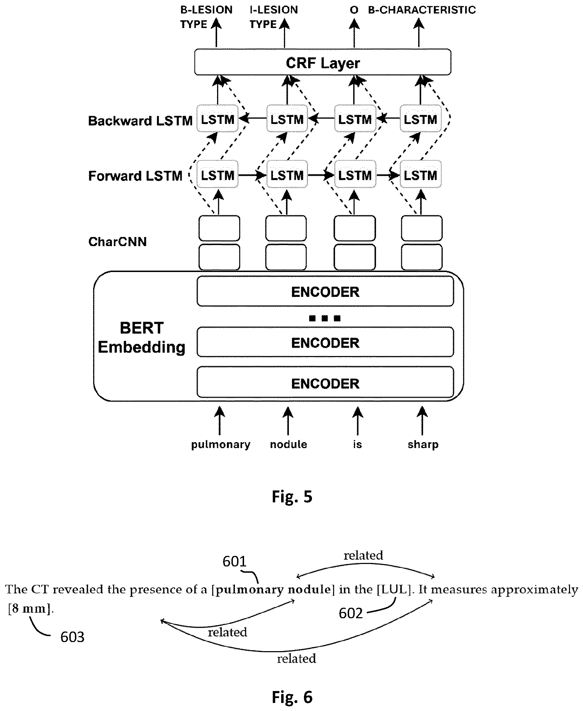

**Figure 5** shows a schematic representation of an embodiment of the NER method.

NER involves the identification of specific attributes related to pulmonary nodules in medical reports. In an embodiment, the attributes identified through NER include Location1 (Absolute Location), Location2 (Relative Location), Lesion Type, Size, Characteristics, Quantity, and Negative. An example of the complete NER architecture is represented in **Figure 5.** The process of NER is important for extracting structured information from unstructured medical text, enabling more efficient data analysis. To accurately perform NER, the text is first tokenized, breaking down the sentences into individual words. For instance, the sentence "The patient has a pulmonary nodule in the upper right lobe" would be tokenized into ["The", "patient", "has", "a", "pulmonary", "nodule", "in", "the", "upper", "right", "lobe"].

Following tokenization, vectorization is employed, where these tokens are transformed into numerical representations, i.e., a feature embedding. Here, the BERTimbau Large model (Souza et al., 2020) was used as the Encoder-only Transformer Model of **101,** which is pre-trained on a large journalistic corpus. BERT generates context-aware embeddings for each token, ensuring that the meaning of each word is understood in its specific context. Importantly, during the training process, the BERT model itself is preferably not iteratively updated or fine-tuned. Instead, BERT's pre-trained vectors are typically fixed, and the downstream layers, i.e.CharCNN, **103;** BiLSTM neural network, **104;** and, **105,** are trained on top of these embeddings from BERT. This means that BERT provides a robust contextual representation, which the following layers use without altering these base embeddings.

After vectorization, the next step involves classifying these tokens into their respective categories. This is achieved through a combination of a CharCNN, **103,** BiLSTM neural network, **104,** and CRF, **105.** While BERT operates at the word level, CharCNN is used to capture subword and character-level features. This works by breaking down each word into individual characters before processing. The CharCNN processes these characters to recognize patterns within the word, such as prefixes, suffixes, or morphological variants, which are especially important in the medical domain where specific terms might be spelled differently or have common roots. Despite BERT handling words as units, CharCNN helps the model capture finer-grained information that BERT might miss, enhancing the model's ability to recognize medical terminology accurately.

The outputs from the CharCNN, **103,** are then passed into a BiLSTM network, **104,** which processes the sequence of tokens in both forward and backward directions to understand the relationships between tokens across the sentence, allowing the model to consider the entire context of the sentence when making classifications. Finally, the CRF layer, **105,** is applied to ensure that the sequence of tags follows a logical and consistent pattern, improving the accuracy of the tagging process. CRF, **105,** is particularly useful in sequence labelling tasks like NER because it considers the entire sequence of predictions when making a decision. For instance, in the sentence "The patient has no pulmonary nodules in the upper right lobe", the CRF ensures that if "pulmonary" is labelled as part of a lesion type, the subsequent word "nodule" is likely to continue this entity, rather than being labelled inconsistently. The CRF takes into account the context and the probability of certain tag sequences, ensuring that the tags assigned to each token make sense together, resulting in more accurate and coherent tagging.

In an embodiment, one or more of the previous steps are parallelized. In particular, obtaining the identified entities (110) and corresponding attributes from the medical description (101) may be carried out in parallel, using GPUs, for a plurality of medical descriptions. Also, the extracting token embeddings from the medical description (101) using an encoder-only transformer model (102), and the extracting feature embeddings from the medical description (101) using a character convolutional neural network model (103), may be carried out in parallel, using GPUs, for a same medical description. In particular, obtaining candidate lesions (206) and corresponding attributes from the image (201) obtained by medical imaging may be carried out in parallel, using GPUs, for a plurality of medical images. Also, in particular, the matching (207) of the candidate lesions (206) and corresponding attributes with the identified entities (110) and corresponding attributes, may be carried out using a CPU or CPUs. In various embodiments, batch processing may be used for the medical descriptions and/or the medical images.

An important aspect of this NER process is the use of Beginning, Inside, and Outside (BIO) tagging. This method labels each token to indicate whether it is the beginning (B), inside (I), or outside (O) of an entity. For example, in the phrase "pulmonary nodule in the upper right lobe", the tokens might be tagged as ["B-LESION TYPE", "I-LESION TYPE", "O", "O", "B- LOCATION1", "I-LOCATION1", "I-LOCATION1"], helping the model accurately categorize entities composed of multiple words. In this method, any token labelled as outside (O) is not related to any identified entity. In a particular embodiment, the NER model was trained with specific hyperparameters, including a learning rate of 0.001, batch size of 8, and dropout rate of 0.4, to optimize its performance in identifying the medical attributes.

The recognition of attributes is assessed using precision, recall, and F1 metrics, which are defined, respectively as:

$$Precision = \frac{TP}{TP+FP} \qquad (1)$$

$$Recall = \frac{TP}{TP+FN} \qquad (2)$$

$$F1 = 2 \times \frac{Precision \times Recall}{Precision+Recall} \qquad (3)$$

where TP is the True Positive count, FP is the false positive count, and FN is the false positive count. The evaluation is conducted under both strict and relaxed criteria. Under the strict criterion, a correct identification is only recognized if all tokens belonging to an entity are accurately identified. In contrast, the relaxed criterion considers a correct identification if any token within the Ground-Truth (GT) entity is correctly identified, counting it as a True Positive (TP).

**Figure 6** discloses a schematic representation of some of the relations between recognized entities that are established in a relation extraction example sentence, wherein: **601** represents an example of a lesion type attribute; **602** represents a location attribute of the lesion; and **603** represents a lesion size attribute.

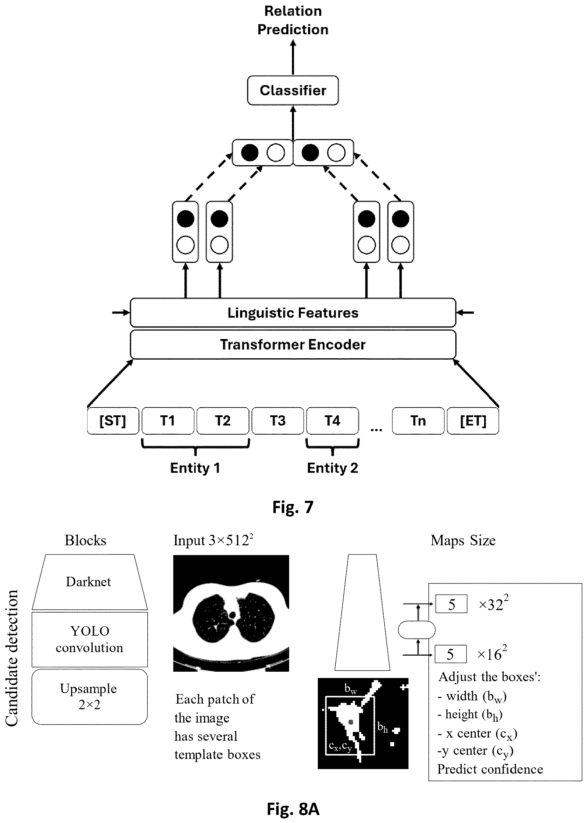

In an embodiment, RE focuses on identifying whether a relationship exists between entities within a given text. For instance, RE might determine whether a "lung nodule" and "upper right lung lobe" mentioned in different parts of a report are related. Another example can be found in **Figure 6,** while the complete RE architecture is represented in **Figure 7.**

In an embodiment, the input to the RE process includes several components: Identified Entities, which are specific terms recognized in the text, such as "nodule" and "upper right lung lobe"; Textual Context, such that the entire document is considered, as entities related to each other may appear in different sentences, meaning that this broader context is crucial for accurately capturing relationships; and Linguistic Features, where Part-of-Speech (POS) tags and dependency parses provide additional syntactic and grammatical information, helping the model understand the structure of the text and how entities are connected.

In an embodiment, the model leverages an encoder-only transformer-based model, **107**, specifically the BERT used in NER, to generate contextual embeddings of tokens. BERT processes the relevant text portions, creating embeddings that represent each token in a high-dimensional space, capturing its nuanced meaning in context. These embeddings are derived from the specific sentences or segments of the text provided - whether a single sentence when both entities are within the same sentence, or multiple sentences when the entities appear in different sentences. This allows BERT to understand how entities fit within their immediate context, which is crucial for accurate RE.

In the RE process, both identified entities and the surrounding textual context are transformed into embeddings, but this happens in slightly different ways:

- Textual Context Embeddings: The relevant sentences or text segments containing the entities are passed through BERT. BERT generates embeddings for every token in these sentences, capturing the contextual meaning based on the surrounding words;
- Entity-Specific Embeddings: The identified entities, such as "nodule" and "upper right lung lobe", are embedded within the same process as the context. These entities receive their own embeddings as part of the overall embedding of the sentence or sentences.

These embeddings from BERT are then combined with linguistic features such as POS tags and dependency parses. The POS tags indicate the grammatical roles of words (e.g., whether a word is a noun or verb), and dependency parses show the syntactical relationships between words (e.g., how a subject relates to a verb). The integration of these features is achieved through concatenation. This means that the BERT embeddings are concatenated with the corresponding vectors representing POS tags and dependency parses, forming a rich, multi-dimensional input for the Neural Network (NN).

In an embodiment, the POS tagger was trained on the Bosque dataset (Rademaker et al., 2017), part of the Universal Dependencies project, providing high-quality linguistic annotations for Portuguese language. The dependency parser is similarly trained on annotated datasets to understand syntactic structures. These tools preprocess the text, producing the necessary linguistic features that are then fed into the RE model along with the BERT embeddings.

At the core of the RE model is a binary classification architecture designed to determine whether a specific relationship exists between entities. Neural networks are employed to perform this classification. The architecture involves feeding the concatenated embeddings (from BERT and linguistic features) into a neural network, **109,** which processes these inputs through multiple layers to output a prediction. This prediction indicates whether a relationship exists between the entities in question, **111.**

During the training phase, the model is exposed to pairs of entities along with their labelled relationships. For example, if a report states "The nodule is located in the upper right lung lobe", the model learns from this example, where "nodule" and "upper right lung lobe" are labelled as related (or not). Entities with the same Identification (ID) are given a relation of "1" (indicating a relationship), while entities with different IDs are given a relation of "0" (indicating no relationship). The neural network adjusts its parameters through supervised learning to minimize prediction errors, ultimately learning to classify relationships accurately in new, unseen data. The evaluation of entity relationships is then performed using accuracy. The accuracy is defined as:

$$\text{Accuracy} = \frac{TP+TN}{TP+FP+TN+FN} \qquad (4)$$

where TN represents the true negative count. For this model, a learning rate of 0.001 and a batch size of 8 were used.

The output of the RE model is a binary classification for each pair of related entities, **111:**

- Relationship Label: A binary label indicates whether a relationship exists ("1") or not ("0");
- Confidence Score: Each prediction is accompanied by a confidence score that indicates the model's certainty regarding the relationship, providing a measure of reliability for the extracted information.

In an embodiment, after the model outputs its predictions, these results can be used to identify connected entities and build relationship trees. This involves associating related entities to clearly map out their connections, allowing for a structured understanding of relationships within the text.

TS is aimed at transforming complex or descriptive expressions into simpler, more canonical forms. The primary objective of this step is to standardize the language, making it more predictable for subsequent phases, such as CV processing.

The simplification process begins with representing both the complex expressions and their desired simplified forms as vectors in a multi-dimensional space. These vectors are generated using BERT word embeddings. Unlike previous steps, the complex expressions in this stage represent only a single token, allowing them to maintain the same dimensional size as the simpler, more predictable forms. For instance, the terms "nodule", "elongated", and "large" could serve as the target simplified forms. Complex phrases such as "nodular finding", "slightly elongated contours", or "of considerable volume" are converted into vectors using the chosen embedding model.

Once these vectors are obtained, the next step involves calculating the similarity between the vector of a complex expression and the vectors of the target simplified forms. This is done using a similarity measure, in particular a cosine similarity, a measure that assesses how close two vectors are by calculating the cosine of the angle between them.

$$S(A,B) = \frac{A \cdot B}{\|A\|\|B\|}$$

(5)

In this equation, $A \cdot B$ represents the dot product of the two vectors A and B, and $\|A\|$ and $\|B\|$ are the magnitudes of vectors A and B, respectively. A cosine similarity value close to 1 indicates that the vectors are nearly identical, while a value closer to 0 suggests that they are orthogonal or unrelated.

After calculating the similarity, in particular a cosine similarity, the expression is replaced with the target form that has the highest similarity score. In other words, the model identifies the most similar vector to the complex expression based on the expected terms and the category of the token identified in the NER phase. For example, since "nodular finding" is found to have a higher cosine similarity with "nodule" than with "mass" and "micronodule", "nodule" is selected as the intended output.

The anatomical segmentation serves as the initial step for the CV module. The module for entity and respective attribute extraction automatically identifies Location1 (Absolute Location) and Location2 (Relative Location), which guides the anatomical segmentation of lung lobes and the filtering of admissible points based on the indicated relative positions (apical/superior, basal/inferior, anterior, posterior, medial, lateral, centrilobular, and juxtapleural/peripheral). The automatic lobe segmentation uses the same method applied for the GT, specifically the approach by Hofmanninger et al.

(2020), which employs a 2D U-Net trained on a slice-by-slice basis with a diverse set of datasets covering various diseases. This segmentation relies directly on the pre-trained algorithm without additional training, achieving a DC of 0.97. For nodules indicated to be in the lingula, all nodules within the LUL and located below the upper boundary of the LLL are considered.

Regarding relative positioning, the segmented regions of the lung lobes are filtered according to Location2, by retaining points within the lobe that fulfil the conditions along the three anatomical axes. For nodules described as peripheral, their position is identified within the outer quarter of each anatomical axis within the lobe, adjacent to the lung walls. Conversely, nodules described as centrilobular are considered to be located in the central region of the lung, encompassing the lobe area up to half the size of the anatomical axes.

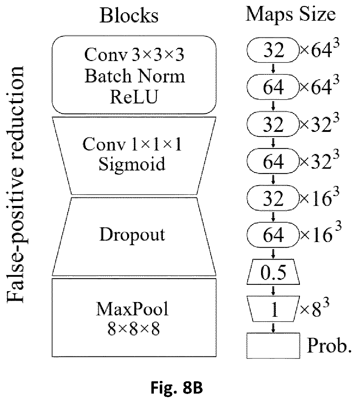

**Figures 8A and 8B** show a schematic representation of an embodiment of the automatic lung nodule detection method, composed of an initial candidate detection followed by a false-positive reduction step.

Following this, lung nodule detection is performed. With the admissible positions, the goal is to obtain lung nodule candidates that can be filtered to closely match what is reported in medical records. The nodule detection used the approach developed in Aresta et al. (2020). The detection module comprises an initial candidate detection phase, followed by a FP reduction step, as is standard in the literature. A figure illustrating this process can be seen in **Figures 8A** and **8B.** The detection algorithm is based on the YOLOv3 architecture (Redmon and Farhadi, 2018) and outputs bounding boxes of potential nodules on the scan.

YOLOv3 is an object detection system that predicts bounding boxes and their labels in a single pass through the network. The feature extraction network generates a feature map F with dimensions $M \times M \times N$, where M represents the grid size and N the number of feature maps. Each grid cell $F_{i,j}$ corresponds to a specific region of the input image, effectively partitioning the image into a grid. Each cell is assigned a set of predefined anchor boxes, defined by their width ($b_w$), height ($b_h$), x-center ($c_x$), and y-center ($c_y$). For each anchor, YOLO predicts the adjustments to these parameters relative to the grid cell, along with a confidence score ($\hat{g}$) that indicates the likelihood of the box containing a nodule. The anchor boxes are optimized using k-means clustering based on the Intersection over Union (IoU) metric, calculated by the following:

$$\text{Intersection over Union} = \frac{TP}{TP+FN+FP}$$

(6)

simplifying the model and ensuring that the anchors align well with the object sizes in the training set. The YOLO network further refines the predefined template boxes across multiple scales, allowing the model to adapt to variations in nodule size and improve detection accuracy.

The network is trained on slices containing nodules by minimizing the detection loss:

$$L_{det} = \sum_{s=1}^{2} \lambda_s \left( \alpha_1 M_{centers} + \alpha_2 M_{dimensions} + \alpha_3 M_{confidence} \right) \qquad (7)$$

where $M_{centers}$, $M_{dimensions}$, and $M_{confidence}$ are the mean square errors associated with the centroid, width/height, and confidence predictions, respectively. The weights $\alpha_i$ and $\lambda_s$ are predefined to balance these components in the overall loss function.

The input to the model is a 512 × 512 × 3 image composed of three neighboring axial CT slices, chosen to leverage 2D feature extractors and benefit from transfer learning with pre-trained networks on natural images. This approach reduces the computational complexity compared to 3D networks while preserving enough volumetric context to help differentiate nodules from other structures, such as blood vessels. The model is trained using the Adam optimizer (Kingma and Ba, 2015), with data augmentation techniques including random crops, rotations, translations, and slight color alterations to maintain the 3D contextual information from the stacked slices. During inference, the model processes the CT volume slice by slice, generating bounding boxes and associated probabilities for nodule presence. To minimize FP, predictions outside the lung regions are excluded, and overlapping predictions are combined by averaging their centroids and retaining the highest confidence score.

The initial candidate detection was trained on the LNDb dataset (Pedrosa et al., 2019) (Ferreira et al., 2024), while the FP reduction step used the LIDC-IDRI dataset (Armato III et al., 2011) to address overfitting to specific acquisition parameters or annotation styles, thereby enhancing the robustness of the entire detection pipeline. This dual-dataset strategy reduces the risk of overestimating performance and facilitates better generalization across varied clinical settings. By targeting a predetermined number of nodules per scan, for example 20 nodules per scan, the approach balances sensitivity with the FP rate, aiming for precise identification through anatomical filtering and nodule characterization. The binary classification of nodule versus non-nodule in the false-positive reduction network was optimized using the binary cross-entropy loss, $\mathcal{L}$, where:

$$\mathcal{L} = -\frac{1}{N} \sum_{i=1}^{N} \sum_{c=1}^{C} y_{i,c} \log\left(\widehat{y_{i,c}}\right) \qquad (8)$$

where $N$ is the number of samples, $C$ represents the number of classes, $y_{i,c}$ corresponds to the ground truth label for instance $i$ in class $c$, and $\widehat{y_{i,c}}$ is the predicted probability for instance $i$ in class $c$.

At that stage, a pool of candidate nodules is identified within the CT images. Following the initial spatial identification, it is necessary to proceed with automated predictions regarding nodule size and characteristics, comparing these predictions with the information provided in the medical reports.

In an embodiment, wherein the size and characteristics are not explicitly mentioned, the default approach is to select the largest nodule. Conversely, if the report refers to a micronodule, it is considered to have a maximum size of up to 6 mm. This criterion aligns with the considerations established for the GT.

The segmentation method employed in this study was the iW-Net, as proposed by (Aresta et al., 2019). This method is based on a 3D U-Net architecture (Ronneberger et al., 2015), consisting of four convolutional layers. The input comprises volumes of 64 × 64 × 64 voxels with a spacing of 1 mm between voxels, and a binarization threshold of 0.5 was applied. The network was trained using the Adam optimizer (Kingma and Ba, 2015) with a learning rate of 0.001 and a batch size of 8 samples. An early stopping criterion was implemented, halting the training after 3 epochs without improvement. The loss function for the U-Net architecture was defined as 1 - IoU. To increase the diversity of the training data, artificial augmentation techniques were applied, including random rotations, translations, flips, and zooms.

In an embodiment, for predicting nodule characteristics, the methodology proposed by Marques et al. (2021) was employed, which is hereby incorporated by reference. This study introduces a multi-task framework for the automated prediction of lung nodule characteristics. Texture is classified into three distinct classes (solid, part-solid, and GGO), while other characteristics are categorized into two classes. The network architecture consists of a CNN with three convolutional layers, which takes as input three parallel axial CT slices centered on the nodule, each with dimensions of 64 × 64 pixels and a resolution of 0.7 mm per pixel. The final activation layer uses a softmax function, with cross-entropy loss guiding the training process, which is defined in equation (8). For binary classifications, a binarization threshold of 0.5 was applied, while for multiclass predictions, the class with the highest probability was selected. Model optimization was conducted using the Nesterov Adam optimizer (Dozat, 2016), with a learning rate of 0.002 and a batch size of 32. An early stopping criterion with a patience of 50 epochs was considered. Data augmentation included vertical and horizontal flips, as well as random rotations from 0° to 360°.

Throughout the matching process, the nodules are rescaled within the range [-1000, 400] Hounsfield Units (HUs). After this rescaling, the automated segmentation and characterization are performed. Subsequently, it is necessary to compare the predicted segmentation with the GT reference to determine the probability that a candidate nodule from the pool is the correct match. A margin of $\pm 3$ mm is allowed relative to the dimensions specified in the medical report to account for measurement variability. This step involves calculating the probability that the true size D falls within the specified range $[D_{min}, D_{max}]$.

The diameter bounds in millimeters are converted to voxel units by first estimating the equivalent ellipsoid volume to better represent the nodule's actual size. This volume is used to translate the nodule into voxel space. The lower and upper voxel limits, $Voxel_{min}$ and $Voxel_{max}$, are determined by dividing the nodule's volume by the volume of a single voxel, accounting for voxel spacing in each dimension.

The probability that the nodule size falls within the desired range is computed using a probability array P derived from segmentation predictions, representing the voxel-wise likelihood of the segmented region corresponding to the nodule. The probability values are flattened into a one-dimensional array, allowing the calculation of the probability that the number of voxels within the segmented region exceeds $Voxel_{min}$ and is less than $Voxel_{max}$.

In an embodiment, to find the probability that the voxel count is within the specified range, the probability of exactly k voxels being included is given by:

$$P(k \text{ voxels}) = \sum_{\text{combinations of } k} \left( \prod_{i \in S_k} P_i \right) \left( \prod_{j \notin S_k} (1 - P_j) \right) \qquad (9)$$

where $S_k$ represents the set of k selected voxels.

The probability that the voxel count lies within the inclusive limits $Voxel_{min}$ and $Voxel_{max}$ is then calculated by summing over the probabilities for all relevant voxel counts:

$$P(Voxel_{min} \leq k \leq Voxel_{max}) = \sum_{k=Voxel_{min}}^{Voxel_{max}} P(k \text{ voxels}) \qquad (10)$$

In an embodiment, this formulation quantifies the likelihood that the segmented region's voxel count lies inclusively within the targeted bounds, effectively integrating over the specified voxel range.

To incorporate the influence of size, a binary presence flag, $f_s$, is set based on whether size information is explicitly referenced in the medical reports:

$$f_s = \begin{cases} 1, & \text{if size information is referenced,} \\ 0, & \text{otherwise.} \end{cases} \qquad (11)$$

For each nodule, the probability of matching specific characteristics, $C_i$, for i = 1, ... , n is also evaluated, where n is the total number of characteristics under study. The presence and relevance of each characteristic are represented by a feature array $f_c(i)$, defined as:

$$f_c(i) = \begin{cases} 1, & \text{if characteristic } C_i \text{ is referenced,} \\ 0, & \text{otherwise,} \end{cases} \qquad (12)$$

and a corresponding weighting array $g_c(i)$ that adjusts the probability based on the values of the characteristics relative to predefined thresholds. These weights, $g_c(i)$, are calculated using rules specific to each characteristic type, ensuring that probabilities reflect the clinical relevance of the characteristic values. When at least one characteristic is present, the array $f_c(i)$ is normalized by dividing by the sum of its elements:

$$f_c(i) = \frac{f_c(i)}{\sum_{i=1}^{n} f_c(i)}, \quad \text{if } \sum_{i=1}^{n} f_c(i) > 0 \tag{13}$$

The overall score $f_{\text{cum}}$ combines the presence flags of both size and characteristics:

$$f_{\text{cum}} = f_s + \left( \sum_{i=1}^{n} f_c(i) > 0 \right) \tag{14}$$

This score $f_{\text{cum}}$ adjusts whether size, characteristics, or both are referenced: if only one is referenced, $f_{\text{cum}} = 1$; if both are referenced, $f_{\text{cum}} = 2$. The combined probability for nodule selection is then defined as:

$$P(N) = \frac{1}{f_{\text{cum}}} \left( P(D_{\min} \leq D \leq D_{\max} \mid S) + \sum_{i=1}^{n} f_c(i) \cdot g_c(i) \right) \tag{15}$$

where S represents the segmentation prediction of the nodule in voxel space. Dividing by $f_{\text{cum}}$ ensures that the influence of the probabilities is normalized based on the presence of either or both references. The candidate nodule with the highest overall probability P(N) is selected as the most likely match. This probabilistic approach ensures that both size and characteristic data are effectively considered, optimizing the identification of the correct nodule within the candidate pool.

For the evaluation, two scenarios were considered: the perfect scenario, in which the module for entity and respective attribute extraction operates perfectly, allowing the performance of the CV module to be assessed independently; and the real scenario, where the prediction results from the module for entity and respective attribute extraction, which may include some identification errors, are passed to the CV module. In both scenarios, precision and recall were used to determine the number of nodules correctly identified in relation to the GT. Additionally, undetected nodules were examined, along with hypothetical nodules that were considered due to errors from the module for entity and respective attribute extraction. The impact on follow-up recommendations was also assessed using Fleischner guidelines in both scenarios, to evaluate whether different nodules per patient lead to varying clinical recommendations. Results demonstrated that the disclosure is effective for detecting and annotating lesions from an image obtained by medical imaging.

The disclosure thus includes associating NER entities related to the same nodule. It also includes a method for Pulmonary Nodule Analysis in chest CT medical reports.

The disclosure includes the creation of annotated image data for computer vision purposes [development of software tools and platforms for generating annotated image datasets; automating the annotation process using NLP and CV techniques; offering data annotation services to healthcare institutions and research organizations].

The disclosure includes restoring imaging findings for image revisit purposes [software solutions integrated into existing PACS/RIS; implementing algorithms to match historical text reports with current imaging data; providing integration services for hospitals and clinics].

The disclosure also includes gathering what was reported textually in a follow-up image for the purpose of perceiving the evolution of the pathological finding [development of clinical decision support systems; utilizing processing to interpret follow-up reports; creating a subscription-based service for healthcare providers].

A computer data processor, as used herein, refers to any system, device, or apparatus capable of processing data in accordance with the methods described in this disclosure. The computer data processor may include one or more processors, such as a central processing unit (CPU), a graphics processing unit (GPU), or any combination thereof. These processors may be implemented as a single chip, a multi-core processor, a distributed computing system, or any other suitable configuration. For example, this can be a central processing unit (CPU), such as an Intel® Core™ i7 processor, and memory modules, including 16 GB of DDR4 RAM. The system may include a solid-state drive (SSD) for storage, one or more optional GPUs (e.g., NVIDIA® GeForce RTX™ 3060), and runs a standard operating system, such as Microsoft® Windows® or Linux®. For example, this can be an embedded system utilizing a microcontroller, such as the ARM® Cortex®-M4 processor, with onboard memory (e.g., 1 MB of flash storage and 256 KB of SRAM). For example, this can also be a cloud-based virtual machine hosted on a server infrastructure, such as an Amazon Web Services (AWS) EC2 instance, featuring virtual CPUs (vCPUs) based on Intel® Xeon® or AMD EPYC™ processors. The instance can be configured for example with 32 GB of RAM, 1 TB of elastic block storage (EBS), and executes server-side software designed to perform the computational processes disclosed in this specification.

The term "comprising" whenever used in this document is intended to indicate the presence of stated features, integers, steps, components, but not to preclude the presence or addition of one or more other features, integers, steps, components, or groups thereof.

The disclosure should not be seen in any way restricted to the embodiments described and a person with ordinary skill in the art will foresee many possibilities to modifications thereof. The above-described embodiments are combinable.

The following claims further set out particular embodiments of the disclosure.

References

(Kirillov et al., 2023) Alexander Kirillov, Eric Mintun, Nikhila Ravi, Hanzi Mao, Chloe Rolland, Laura Gustafson, Tete Xiao, Spencer Whitehead, Alexander C Berg, Wan-Yen Lo, et al. Segment anything. In Proceedings of the IEEE/CVF International Conference on Computer Vision, pages 4015-4026, 2023. doi: 10.1109/ICCV51070.2023.00371.

(Radford et al., 2021) Alec Radford, Jong Wook Kim, Chris Hallacy, Aditya Ramesh, Gabriel Goh, Sandhini Agarwal, Girish Sastry, Amanda Askell, Pamela Mishkin, Jack Clark, Gretchen Krueger, and Ilya Sutskever. Learning transferable visual models from natural language supervision. In Marina Meila and Tong Zhang, editors, Proceedings of the 38th International Conference on Machine Learning, volume 139 of Proceedings of Machine Learning Research, pages 8748-8763. PMLR, 18-24 Jul 2021. URL https://proceedings.mlr.press/v139/radford21a.html.

(Cheng et al., 2024) Tianheng Cheng, Lin Song, Yixiao Ge, Wenyu Liu, Xinggang Wang, and Ying Shan. Yoloworld: Real-time open-vocabulary object detection. In Proceedings of the IEEE/CVF Conference on Computer Vision and Pattern Recognition, pages 16901-16911, 2024. URL https://openaccess.thecvf.com/content/CVPR2024/papers/Cheng_YOLO-World_Real-Time_OpenVocabulary_Object_Detection_CVPR_2024_paper.pdf.

(Souza et al., 2020) Fábio Souza, Rodrigo Nogueira, and Roberto Lotufo. Bertimbau: pretrained bert models for brazilian portuguese. In Intelligent Systems: 9th Brazilian Conference, BRACIS 2020, Rio Grande, Brazil, October 20-23, 2020, Proceedings, Part I 9, pages 403-417. Springer, 2020. doi: 10.1007/978-3-030-61377-8_28.

(Rademaker et al., 2017) Alexandre Rademaker, Fabricio Chalub, Livy Real, Cláudia Freitas, Eckhard Bick, and Valeria de Paiva. Universal dependencies for portuguese. In Proceedings of the Fourth International Conference on Dependency Linguistics (Depling), pages 197-206, Pisa, Italy, September 2017. URL http://aclweb.org/anthology/W17-6523.

(Hofmanninger et al., 2020) Johannes Hofmanninger, Forian Prayer, Jeanny Pan, Sebastian Röhrich, Helmut Prosch, and Georg Langs. Automatic lung segmentation in routine imaging is primarily a data diversity problem, not a methodology problem. European Radiology Experimental, 4(1):1-13, 2020. doi: 10.1186/s41747-020-00173-2.

(Aresta et al., 2020) Guilherme Aresta, Carlos Ferreira, Joao Pedrosa, Teresa Araújo, Joao Rebelo, Eduardo Negrao, Margarida Morgado, Filipe Alves, Antonio Cunha, Isabel Ramos, and Aurelio Campilho. Automatic Lung Nodule Detection Combined With Gaze Information Improves Radiologists' Screening Performance. IEEE journal of biomedical and health informatics, 24(10):2894-2901, 2020. ISSN 21682208. doi: 10.1109/JBHI.2020.2976150.

(Redmon and Farhadi, 2018) Joseph Redmon and Ali Farhadi. Yolov3: An incremental improvement. CoRR, abs/1804.02767, 2018. URL http://arxiv.org/abs/1804.02767.

(Kingma and Ba, 2015) Diederik P. Kingma and Jimmy Ba. Adam: A method for stochastic optimization. Computing Research Repository (CoRR), abs/1412.6980, 2015. doi: 10.48550/arXiv.1412.6980.

(Pedrosa et al., 2019) João Pedrosa, Guilherme Aresta, Carlos Ferreira, Márcio Rodrigues, Patricia Leitão, André Silva Carvalho, João Rebelo, Eduardo Negrão, Isabel Ramos, António Cunha, et al. LNDb: a lung nodule database on computed tomography. Preprint at https://arxiv.org/abs/1911.08434, 2019.

(Ferreira et al., 2024) Carlos A Ferreira, Célia Sousa, Inês Dias Marques, Pedro Sousa, Isabel Ramos, Miguel Coimbra, and Aurélio Campilho. LNDb v4: pulmonary nodule annotation from medical reports. Scientific Data, 11(1):512, 2024b. doi: 10.1038/s41597-024-03345-6.

(Armato III et al., 2011) Samuel G Armato III, Geoffrey McLennan, Luc Bidaut, Michael F McNitt-Gray, Charles R Meyer, Anthony P Reeves, Binsheng Zhao, Denise R Aberle, Claudia I Henschke, Eric A Hoffman, et al. The Lung Image Database Consortium (LIDC) and Image Database Resource Initiative (IDRI): A Completed Reference Database of Lung Nodules on CT Scans. Medical Physics, 38 (2):915, 2011. ISSN 00942405. doi: 10.1118/1.3528204.

(Aresta et al., 2019) Guilherme Aresta, Colin Jacobs, Teresa Araújo, António Cunha, Isabel Ramos, Bram van Ginneken, and Aurélio Campilho. iW-Net: an automatic and minimalistic interactive lung nodule segmentation deep network. Scientific reports, 9(1):1-9, 2019. doi: 10.1038/s41598-019-48004-8.

(Ronneberger et al., 2015) Olaf Ronneberger, Philipp Fischer, and Thomas Brox. U-net: Convolutional networks for biomedical image segmentation. In Medical image computing and computer-assisted intervention-MICCAI 2015: 18th international conference, Munich, Germany, October 5-9, 2015, proceedings, part III 18, pages 234-241. Springer, 2015. doi: 10.1007/978-3-319-24574-4_28.

(Marques et al., 2021) Sonia Marques, Filippo Schiavo, Carlos A Ferreira, Joao Pedrosa, Antonio Cunha, and Aurelio Campilho. A multi-task CNN approach for lung nodule malignancy classification and characterization. Expert Systems with Applications, 184:115469, 2021. doi: 10.1016/j.eswa.2021.115469.

(Dozat, 2016) Timothy Dozat. Incorporating Nesterov momentum into Adam. In Proceedings of the 4th International Conference on Learning Representations, pages 1-4, 2016. URL https://openreview.net/pdf/OM0jvwB8jlp57ZJjtNEZ.pdf.

**Claims**

1. A computer-implemented method for detecting and annotating lesions from an image (201) obtained by medical imaging, and from a corresponding medical description (101) comprising identified entities of medical lesions, the method comprising the steps:

   obtaining identified entities (110) and corresponding attributes from the medical description (101) using one more machine-learning models;
   obtaining candidate lesions (206) and corresponding attributes from the image (201) obtained by medical imaging using one more machine-learning models;
   matching (207) the candidate lesions (206) and corresponding attributes with the identified entities (110) and corresponding attributes; and
   selecting the matched filtered candidate lesions and identified entities (208) using a highest probability criterion corresponding to an entity identified in the medical description (101) matching a candidate lesion obtained from the medical image (201).

2. Method according to the previous claim wherein the highest probability criterion comprises corresponding attributes of an entity identified in the medical description (101) with matching attributes of a candidate lesion obtained from the medical image (201), wherein said attributes comprise size.

3. Method according to the previous claim wherein matching size comprises integrating a probability of voxels of a candidate lesion in the medical image (201) matching an entity identified in the medical description (101).

4. Method according to any of the previous claims wherein selecting the matched filtered candidate lesions and identified entities (208) using a highest probability criterion is independently carried out for each anatomical region of a plurality of anatomical regions present in the medical image (201) and/or present in the medical description (101).

5. Method according to the previous claim wherein the plurality of anatomical regions is obtained by segmentation of the medical image (201).

6. Method according to any of the previous claims wherein obtaining the identified entities (110) and corresponding attributes from the medical description (101), comprises the steps of:

   extracting token embeddings from the medical description (101) using an encoder-only transformer model (102);
   extracting feature embeddings from the medical description (101) using a character convolutional neural network model (103);
identifying entities from the extracted token embeddings and from the extracted feature embeddings using a bidirectional long short-term memory neural network (104) followed by a conditional random field model (105), to obtain identified entities (106) and respective attributes, said attributes comprising a location of each identified entity;
extracting text segments (107) from the medical description (101) for each of the identified entities (106, 110), where the text segment includes the identified entity;
extracting token embeddings from the extracted text segments (107) using an encoder-only transformer model (108) and tagging the identified entities with a tagging model (108) to classify relationships between said attributes; and
pairing the identified entities (111) using a probability model (109).

7. Method according to the previous claim wherein the tagging of the identified entities with a tagging model (108) comprises assigning attributes denoting beginning, inside, or outside of a text segment consisting of an identified entity.

8. Method according to any of the previous claims wherein obtaining candidate lesions (206) and corresponding attributes from the image (201) obtained by medical imaging, comprises the steps of:

    segmenting (203) the medical image (201) into image segments, wherein the image segments comprise the locations of the identified entities (202);
    obtaining candidate lesions and corresponding attributes (206) from the image segments using a convolution neural network (204); and
    filtering candidate lesions using a false positive reduction model (205) for removing false positive candidate lesions.

9. Method according to any of the previous claims wherein a lesion is a damage or abnormal change in a tissue.

10. The method according to according to any of the previous claims, wherein the attributes of the identified entities (110) from the medical description (101) comprise at least one of: a first location related to an absolute lesion location; a second location related to a relative lesion location; a lesion type; a lesion size attribute; anatomical lesion characteristic or characteristics; a lesion quantity attribute; and/or a non-appearance attribute.

11. The method according to any of the previous claims, comprising a subsequent step of annotating the medical image (201) with selected matched filtered candidate lesions and identified entities (209).

12. The method according to according to any of the previous claims, wherein the encoder-only transformer model (108) is a Bidirectional Encoder Representations from Transformers, BERT, model.

13. A data processing device configured for carrying out the steps of the method according to any of the previous claims.

14. A computer program comprising instructions which, when the program is executed by a computer, cause the computer to carry out the steps of the method according to any of the previous claims.

15. A computer-readable storage medium comprising instructions which, when executed by a computer, cause the computer to carry out the steps of the method according to any of the previous claims, in particular the computer-readable storage medium being a non-transitory computer-readable storage medium.

Fig. 1

**Fig. 2A**

**Fig. 2B**

**NER**

The patient has a history of heavy smoking. One very rounded nodular finding is seen in the LLL measuring 5mm. No other nodules are seen.

The patient has a history of heavy smoking. One very rounded nodular finding is seen in the LLL measuring 5 mm. No other nodules are seen.

**RE**

The patient has a history of heavy smoking. One very rounded nodular finding is seen in the LLL measuring 5 mm. No other nodules are seen.

**TS**

ID1:
- Characteristic: Round;
- Lesion Type: Nodule;
- Location1: LLL;
- Size: 5 mm.

**Fig. 3**

401 402 406 403

ID1:
- Location1: LLL.

**?** Prob. = 0.6

**?** Prob. = 0.8

404

ID1:
- Characteristic: Round;
- Size: 5 mm.

**X** 1 mm, linear

✓ 5 mm, round

405

**Fig. 4**

B-LESION   I-LESION         O   B-CHARACTERISTIC
TYPE       TYPE

CRF Layer

Backward LSTM  LSTM ← LSTM ← LSTM ← LSTM

Forward LSTM   LSTM → LSTM → LSTM → LSTM

CharCNN

BERT
Embedding

ENCODER

■ ■ ■

ENCODER

ENCODER

pulmonary   nodule   is   sharp

**Fig. 5**

601

related

The CT revealed the presence of a [pulmonary nodule] in the [LUL]. It measures approximately
[8 mm].

602

603

related

related

**Fig. 6**

**Relation
Prediction**

**Classifier**

**Linguistic Features**

**Transformer Encoder**

[ST] | T1 | T2 | T3 | T4 | ... | Tn | [ET]

**Entity 1**          **Entity 2**

**Fig. 7**

Blocks          Input $3 \times 512^2$          Maps Size

Candidate detection

Darknet

YOLO
convolution

Upsample
$2 \times 2$

Each patch of
the image
has several
template boxes

$b_w$
$b_h$
$c_x, c_y$

5  $\times 32^2$

5  $\times 16^2$

Adjust the boxes':
- width ($b_w$)
- height ($b_h$)
- x center ($c_x$)
-y center ($c_y$)
Predict confidence

**Fig. 8A**

Fig. 8B

Europäisches
Patentamt

European
Patent Office

Office européen
des brevets

# EUROPEAN SEARCH REPORT

**Application Number**

EP 25 16 6487

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 113 485 555 A (SHANGHAI UNITED IMAGING INTELLIGENCE HEALTHCARE CO LTD) 8 October 2021 (2021-10-08) | 1-5, 8-11, 13-15 | INV. G06V10/82 G16H30/40 |
| A | * paragraph [0001] - paragraph [0134]; figures 1-8 * | 6,7,12 | G16H50/20 |
| A | US 2023/068201 A1 (ICHINOSE AKIMICHI [JP]) 2 March 2023 (2023-03-02) * paragraph [0043] - paragraph [0133]; figures 1-18 * | 1-15 | |

TECHNICAL FIELDS
SEARCHED (IPC)

G06V
G16H

The present search report has been drawn up for all claims

| Place of search | Date of completion of the search | Examiner |
|---|---|---|
| The Hague | 23 May 2025 | Thibault, Guillaume |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 16 6487

This annex lists the patent family members relating to the patent documents cited in the above-mentioned European search report.
The members are as contained in the European Patent Office EDP file on
The European Patent Office is in no way liable for these particulars which are merely given for the purpose of information.

23-05-2025

| Patent document cited in search report | | Publication date | Patent family member(s) | | Publication date |
|---|---|---|---|---|---|
| CN 113485555 | A | 08-10-2021 | NONE | | |
| US 2023068201 | A1 | 02-03-2023 | JP | 2023034089 A | 13-03-2023 |
| | | | US | 2023068201 A1 | 02-03-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ALEXANDER KIRILLOV** ; **ERIC MINTUN** ; **NIKHILA RAVI** ; **HANZI MAO** ; **CHLOE ROLLAND** ; **LAURA GUSTAFSON** ; **TETE XIAO** ; **SPENCER WHITEHEAD** ; **ALEXANDER C BERG** ; **WAN-YEN LO et al.** Segment anything. *Proceedings of the IEEE/CVF International Conference on Computer Vision*, 2023, 4015-4026
- Learning transferable visual models from natural language supervision. **ALEC RADFORD** ; **JONG WOOK KIM** ; **CHRIS HALLACY** ; **ADITYA RAMESH** ; **GABRIEL GOH** ; **SANDHINI AGARWAL** ; **GIRISH SASTRY** ; **AMANDA ASKELL** ; **PAMELA MISHKIN** ; **JACK CLARK**. Proceedings of the 38th International Conference on Machine Learning. 18 July 2021, vol. 139, 8748-8763
- **TIANHENG CHENG** ; **LIN SONG** ; **YIXIAO GE** ; **WENYU LIU** ; **XINGGANG WANG** ; **YING SHAN**. Yoloworld: Real-time open-vocabulary object detection. *Proceedings of the IEEE/CVF Conference on Computer Vision and Pattern Recognition*, 2024, 16901-16911, https://openaccess.thecvf.com/content/CVPR2024/papers/Cheng_YOLO-World_Real-Time_OpenVocabulary_Object_Detection_CVPR_2024_paper.pdf
- Bertimbau: pretrained bert models for brazilian portuguese. **FÁBIO SOUZA** ; **RODRIGO NOGUEIRA** ; **ROBERTO LOTUFO**. Intelligent Systems: 9th Brazilian Conference, BRACIS 2020, Rio Grande, Brazil. Springer, 20 October 2020, vol. 9, 403-417
- **ALEXANDRE RADEMAKER** ; **FABRICIO CHALUB** ; **LIVY REAL** ; **CLÁUDIA FREITAS** ; **ECKHARD BICK** ; **VALERIA DE PAIVA**. Universal dependencies for portuguese. *Proceedings of the Fourth International Conference on Dependency Linguistics (Depling)*, September 2017, 197-206, http://aclweb.org/anthology/W17-6523
- **JOHANNES HOFMANNINGER** ; **FORIAN PRAYER** ; **JEANNY PAN** ; **SEBASTIAN RÖHRICH** ; **HELMUT PROSCH** ; **GEORG LANGS**. Automatic lung segmentation in routine imaging is primarily a data diversity problem, not a methodology problem. *European Radiology Experimental*, 2020, vol. 4 (1), 1-13

- **GUILHERME ARESTA** ; **CARLOS FERREIRA** ; **JOAO PEDROSA** ; **TERESA ARAÚJO** ; **JOAO REBELO** ; **EDUARDO NEGRAO** ; **MARGARIDA MORGADO** ; **FILIPE ALVES** ; **ANTONIO CUNHA** ; **ISABEL RAMOS**. Automatic Lung Nodule Detection Combined With Gaze Information Improves Radiologists' Screening Performance. *IEEE journal of biomedical and health informatics*, 2020, vol. 24 (10), ISSN 21682208, 2894-2901
- **JOSEPH REDMON** ; **ALI FARHADI**. Yolov3: An incremental improvement. *CoRR, abs/1804.02767*, 2018, http://arxiv.org/abs/1804.02767
- **DIEDERIK P. KINGMA** ; **JIMMY BA**. Adam: A method for stochastic optimization. *Computing Research Repository (CoRR), abs/1412.6980*, 2015
- **JOÃO PEDROSA** ; **GUILHERME ARESTA** ; **CARLOS FERREIRA** ; **MÁRCIO RODRIGUES** ; **PATRICIA LEITÃO** ; **ANDRÉ SILVA CARVALHO** ; **JOÃO REBELO** ; **EDUARDO NEGRÃO** ; **ISABEL RAMOS** ; **ANTÓNIO CUNHA et al.** LNDb: a lung nodule database on computed tomography. *Preprint*, 2019, https://arxiv.org/abs/1911.08434
- **CARLOS A FERREIRA** ; **CÉLIA SOUSA** ; **INÊS DIAS MARQUES** ; **PEDRO SOUSA** ; **ISABEL RAMOS** ; **MIGUEL COIMBRA** ; **AURÉLIO CAMPILHO**. LNDb v4: pulmonary nodule annotation from medical reports. *Scientific Data*, 2024, vol. 11 (1), 512
- **SAMUEL G ARMATO III** ; **GEOFFREY MCLENNAN** ; **LUC BIDAUT** ; **MICHAEL F MCNITT-GRAY** ; **CHARLES R MEYER** ; **ANTHONY P REEVES** ; **BINSHENG ZHAO** ; **DENISE R ABERLE** ; **CLAUDIA I HENSCHKE** ; **ERIC A HOFFMAN et al.** The Lung Image Database Consortium (LIDC) and Image Database Resource Initiative (IDRI): A Completed Reference Database of Lung Nodules on CT Scans. *Medical Physics*, 2011, vol. 38 (2), ISSN 00942405, 915
- **GUILHERME ARESTA** ; **COLIN JACOBS** ; **TERESA ARAÚJO** ; **ANTÓNIO CUNHA** ; **ISABEL RAMOS** ; **BRAM VAN GINNEKEN** ; **AURÉLIO CAMPILHO**. iW-Net: an automatic and minimalistic interactive lung nodule segmentation deep network. *Scientific reports*, 2019, vol. 9 (1), 1-9

- U-net: Convolutional networks for biomedical image segmentation. **OLAF RONNEBERGER** ; **PHILIPP FISCHER** ; **THOMAS BROX**. Medical image computing and computer-assisted intervention-MICCAI 2015: 18th international conference, Munich, Germany. Springer, 05 October 2015, vol. 18, 234-241

- **SONIA MARQUES** ; **FILIPPO SCHIAVO** ; **CARLOS A FERREIRA** ; **JOAO PEDROSA** ; **ANTONIO CUNHA** ; **AURELIO CAMPILHO**. A multi-task CNN approach for lung nodule malignancy classification and characterization. *Expert Systems with Applications*, 2021, vol. 184, 115469
- **TIMOTHY DOZAT**. Incorporating Nesterov momentum into Adam. *Proceedings of the 4th International Conference on Learning Representations*, 2016, 1-4, https://openreview.net/pdf/OM0jvwB8jlp57ZJjtNEZ.pdf